# EUROPÄISCHE PATENTSCHRIFT

(11) **EP 0 636 409 B1**
(45) Veröffentlichungstag und Bekanntmachung des Hinweises auf die Patenterteilung: **08.10.1997**
(21) Anmeldenummer: 94111539.6
(22) Anmeldetag: 23.07.1994
(51) Int. Cl.: B01J 27/185, B01J 27/187, B01J 37/08

(54) **Kobaltkatalysatoren**
Cobalt catalysts
Catalyseurs au cobalt

(30) Priorität: 31.07.1993 DE 4325847
(43) Veröffentlichungstag der Anmeldung: 01.02.1995
(73) Patentinhaber: BASF Aktiengesellschaft, 67063 Ludwigshafen (DE)
(72) Erfinder: Breitscheidel, Boris, Dr., D-36043 Fulda (DE); Polanek Peter, Dr., D-69469 Weinheim (DE); Irgang, Matthias, Dr., D-69121 Heidelberg (DE); Petersen, Hermann, Dr., D-67269 Gruenstadt (DE); Linden, Gerd, Dr., D-69117 Heidelberg (DE); Voit, Guido, Dr., D-69198 Schriesheim (DE); Witzel, Tom, Dr., D-67069 Ludwigshafen (DE)

(56) Entgegenhaltungen:
- EP-A- 0 383 132

## Beschreibung

Die vorliegende Erfindung betrifft neue Kobaltkatalysatoren, deren katalytisch aktive Masse aus Kobalt, Phosphor, Mangan und Alkali besteht und die durch zwei Calcinierungsschritte bei Endtemperaturen von 550 bis 750°C und 800 bis 1000°C hergestellt werden.

Aus der EP-A-445 589 sind Hydrierkatalysatoren bekannt, deren katalytisch aktive Masse 20 bis 95 Gew.-% Kobaltoxid, 0,5 bis 60 Gew.-% Oxide der Metalle Mangan, Nickel, Eisen, Chrom, Molybdän, Wolfram oder Phosphor und 0,5 bis 20 Gew.-% Oxide der Alkali- oder Erdalkali-, der Seltenen Erdengruppe, Scandium oder Yttrium enthalten.

Aus der DE-A-34 03 377 sind geformte Katalysatormassen bekannt, die metallisches Kobalt- und/oder Nickel, mit einem Gehalt von weniger als 0,1 Gew.-% an Alkali- und/oder Erdalkalioxiden, enthalten und die bei Temperaturen von kleiner oder gleich 500°C durch Reduktion hergestellt wurden. Die geformten Katalysatormassen haben eine Druckhärte von mehr als 300 kp/cm².

Die genannten Katalysatoren haben jedoch den Nachteil, daß sie nicht ausreichend basenstabil sind um eine lange Lebensdauer in basischem Medium zu gewährleisten.

Der vorliegenden Erfindung lag daher die Aufgabe zugrunde, den zuvor genannten Nachteilen abzuhelfen.

Demgemäß wurden neue und verbesserte Kobaltkatalysatoren, deren katalytisch aktive Masse aus 55 bis 98 Gew.-% Kobalt, 0,2 bis 15 Gew.-% Phosphor, 0,2 bis 15 Gew.-% Mangan und 0,2 bis 15 Gew.-% Alkali, berechnet als Oxid, besteht, gefunden, welche dadurch gekennzeichnet sind, daß man die Katalysatormasse in einem ersten Schritt bei Endtemperaturen von 550 bis 750°C und in einem zweiten Schritt bei Endtemperaturen von 800 bis 1000°C calciniert und die Kobaltkatalysatoren eine spezifische Oberfläche von größer oder gleich 1,5 m²/g aufweisen sowie ein Verfahren zur Hydrierung von organischen Nitrilen und/oder Iminen, durch Einsatz der neuen Kobaltkatalysatoren.

Die katalytisch aktive Masse der erfindungsgemäßen Kobaltkatalysatoren besteht aus 55 bis 98 Gew.-% Kobalt, bevorzugt 75 bis 95 Gew.-% Kobalt, besonders bevorzugt 85 bis 95 Gew.-% Kobalt, 0,2 bis 15 Gew.-% Phosphor, bevorzugt 0,5 bis 10 Gew.-% Phosphor, besonders bevorzugt 0,5 bis 5 Gew.-% Phosphor, 0,2 bis 15 Gew.-% Mangan, bevorzugt 2 bis 10 Gew.-% Mangan, besonders bevorzugt 3 bis 7 Gew.-% Mangan und 0,2 bis 15 Gew.-% Alkali, bevorzugt 0,5 bis 5 Gew.-% Alkali, besonders bevorzugt 0,5 bis 4 Gew.-% Alkali, berechnet als Oxid.

Als Alkali eignen sich bevorzugt Lithium, Natrium, Kalium und/oder Cäsium, besonders bevorzugt Natrium und/oder Kalium.

Die erfindungsgemäßen Kobaltkatalysatoren lassen sich wie folgt herstellen:

Eine Lösung eines Kobalt-Salzes, bevorzugt eines anorganischen Kobalt-Salzes und gegebenenfalls der gewünschten Promotoren Mangan, Phosphor und/oder Alkalimetalle in Form ihrer wasserlöslichen Salze (im Normalfall pH-Wert < 7) kann durch Zugabe einer alkalischen Lösung eine Mischung der im Katalysator enthaltenen Hauptbestandteile in Form der Carbonate, Hydroxide oder Oxide ausgefällt werden. Die alkalische Lösung kann durch Lösen von z.B. Alkalicarbonaten oder -hydroxiden, Ammoniak, Ammoniumcarbonat oder Ammoniumhydrogencarbonat oder ähnlichen basischen Salzen in Wasser hergestellt werden. Die Konzentrationen sowohl der Metallsalz - als auch der Fällösung - sollten so eingestellt werden, daß die resultierende Fällmaische noch gerührt werden kann. Werden die Promotoren nicht in diesem Schritt mitgefällt, können sie in einer der weiter unten beschriebenen Verfahrensstufen zugefügt werden. Die Zugabe der basischen Lösung wird solange fortgesetzt, bis eine vollständig Ausfällung erreicht ist. Das Produkt der Fällung kann erforderlichenfalis nachgerührt, mit üblichen technischen Mitteln abfiltriert und von unerwünschten wasserlöslichen Fremdionen freigewaschen werden.

Der so entstandene Filterkuchen kann dann bei Temperaturen von 50 bis 200°C getrocknet und das so erhaltene Gut vermahlen werden. Alternativ ist eine Aufschlämmung und ein anschließendes Versprühen der Maische in einem Sprühturm möglich. Hierbei entsteht bei Temperaturen zwischen 100 und 600°C ein Sprühpulver. Wird das Versprühen gewählt, so können dem Katalysator auch in diesem Verfahrensschritt die Promotoren Mangan, Phosphor und/oder Alkalimetalle in Form ihrer Salze beigefügt werden.

Die so erzeugten Pulver können calciniert werden. Die calcinierten Pulver können in verschiedener Weise zu Formkörpern verformt werden. So ist es möglich, die Pulver zu tablettieren, zu extrudieren oder mit Hilfe einer Strangpresse zu Strängen bestimmter Form und Größe zu verpressen. In allen Fällen können dem Pulver Verformungshilfsmittel wie Grafit oder Stearinsäure beigemischt werden.

Die Calcinierung wird in mindestens zwei Calzinierschritten durchgeführt, wobei ein Calcinierschritt bei Endtemperaturen von 550 bis 750°C und ein anderer Calcinierschritt bei Endtemperaturen von 800 und 1000°C folgt. Es können weitere ein- oder mehrstufige thermische Behandlungen bei Temperaturen von 200 bis 1100°C, bevorzugt 400 bis 1000°C, besonders bevorzugt 500 bis 900°C durchgeführt werden.

Man erhält dadurch Kobaltkatalysatoren mit einer Oberfläche von größer oder gleich 1,5 m²/g, also 1,5 bis 500 m²/g, bevorzugt 5 bis 300 m²/g, besonders bevorzugt 10 bis 250 m²/g.

Als Formen sind alle geometrischen Körper herstellbar, die sich in Festbettreaktoren einfüllen lassen.

Es eignen sich sowohl Voll- als auch Trägerkatalysatoren für Hydrierungen. Dies gilt auch für die Umsetzung von Nitrilen und Iminen mit Wasserstoff zu den entsprechenden Aminen.

Als Kriterium der Langzeitstabilität (z.B. auch der Basenstabilität) wurde ein Test entwickelt, der es erlaubt, schon nach kurzer Zeit eine Aussage über das Standzeitverhalten eines Katalysators unter Reaktionsbedingungen zu treffen.

Dieser als Kochtest bezeichnete Kurztest kann folgendermaßen durchgeführt werden:

Ein mit Wasserstoff oberhalb von 250°C reduzierter Katalysator und eine wäßrige Base wie NaOH oder KOH können in einen Autoklaven unter Inertgasatmosphäre eingefüllt und bei ca. 160°C unter autogenem Druck von ca. 5 bar 12 h gehalten werden. Nach Abkühlen, Abdekantieren der Flüssigkeit, Waschen des Katalysators mit Wasser kann die Härte unter Inertgasatmospäre z.B. unter Stickstoff ermittelt werden.

Ein Katalysator mit einer Schneidhärte von größer oder gleich 10 Newton, also 10 bis 1000 Newton, bevorzugt 15 bis 100 Newton, besonders bevorzugt 20 bis 50 nach dem Kochtest besitzt in der Regel eine ausreichende Langzeitstabilität (Standzeit > 3 000 h).

Die erfindungsgemäßen Kobaltkatalysatoren eignen sich als Hydrierkatalysatoren, besonders für Umsetzungen von Nitrilen mit Wasserstoff zu primären Aminen.

### Beispiele

### Kochtest

In einem 250 ml Autoklaven mit Tefloneinsatz wurden 10 ml Katalysator in reduzierter Form unter Stickstoff und 100 ml einer 2,5%igen wässrigen NaOH Lösung eingefüllt. Die Reduktion des Katalysators wurde zuvor in einer kontinuierlichen Apparatur mit H₂ bei 360°C innerhalb von 5 h durchgeführt. Der verschlossene Autoklav wurde auf 160°C aufgeheizt. Dabei entstand ein autogener Druck von ca. 5 bar. Die Temperatur wurde für 12 h auf 160°C gehalten, nach dem Abkühlen die Flüssigkeit abdekantiert, der Katalysator mit Wasser gewaschen und anschließend die Härte unter N₂ ermittelt.

### Katalysatorherstellung

Die Angaben der Gewichtsprozente beziehen sich auf die jeweiligen Oxide im geglühten Katalysator, der Phosphor-Gehalt ist als H₃PO₄ angegeben.

### Katalysator A

Durch Auflösen von Kobaltnitrat, Mangannitrat und Phosphorsäure in Wasser wurde eine Lösung hergestellt, die 10 Gew.-% Kobalt, 0,55 Gew.-% Mangan und 0,45 Gew.-% H₃PO₄ enthält. Durch Zugabe einer 20%igen Natriumcarbonatlösung wurde bei einer Temperatur von 50°C gefällt. Der entstandene Niederschlag wurde gewaschen, bis im Waschwasser kein Natrium oder Nitrat mehr nachweisbar war. Der so erhaltene Feststoff wurde mit Wasser angemaischt und in einem Sprühturm versprüht (Eingangstemperatur = 550°C). Das Sprühgut wurde bei 500°C getrocknet, gekollert und im Extruder zu Strängen von 4 mm Durchmesser verformt. Die Stränge wurden bei 100 bis 120°C getrocknet und anschließend 1 h bei 650°C und danach 3 h bei 850°C calziniert.

Der so hergestellte Katalysator enthielt 90,4 Gew.-% Kobalt, 5,1 Gew.-% Mangan, 0,3 Gew.-% Natrium und 3,1 Gew.-% phosphor mit einer spezifischen Oberfläche von 1,6 m²/g.

### Katalysator B

Die Herstellung erfolgte analog Katalysator A, jedoch wurden vor der Verstrangung zu je 4 kg Sprühpulver 73 g H₃PO₄ zugemischt. Der Katalysator wurde 1 h bei 650°C und danach 3 h bei 820°C calziniert. Man erhielt einen Katalysator mit einer spezifischen Oberfläche von 3,2 m²/g und folgender chemischer Zusammensetzung: 89,5 Gew.-% Kobalt, 5,05 Gew.-% Mangan, 1,41 Gew.-% Natrium, 4,5 Gew.-% Phosphor.

### Katalysator C

Die Herstellung erfolgte analog Katalysator A, jedoch wurden vor der Verstrangung zu je 4 kg Sprühpulver 19,8 g NaOH zugemischt. Der Katalysator wurde 1 h bei 650°C und danach 1 h bei 800°C calziniert. Man erhielt einen Katalysator mit einer spezifischen Oberfläche von 15,1 m²/g und folgender chemischer Zusammensetzung: 89,9 Gew.-% Kobalt, 5,1 Gew.-% Mangan, 1,9 Gew.-% Natrium, 4,2 Gew.-% Phosphor.

### Katalysator D

Die Herstellung erfolgte analog Katalysator A, jedoch wurden vor der Verstrangung zu je 4 kg Sprühpulver 12,8 g H₃PO₄ zugemischt. Der Katalysator wurde 1 h bei 650°C und danach 3 h bei 800°C calciniert. Man erhielt einen Katalysator mit einer spezifischen Oberfläche von 3,0 m²/g und folgender chemischer Zusammensetzung: 90,4 Gew.-% Kobalt, 5,1 Gew.-% Mangan, 1,36 Gew.-% Natrium, 2,8 Gew.-% Phosphor.

### Katalysator E

Die Herstellung erfolgte analog Katalysator A, jedoch wurden vor der Verstrangung zu je 4 kg Sprühpulver 18,3 g NaOH zugemischt. Der Katalysator wurde 1 h bei 650°C und danach 2 h bei 850°C calciniert. Man erhielt einen Katalysator mit einer spezifischen Oberfläche von 6,2 m²/g und folgender chemischer Zusammensetzung: 89,9 Gew.-% Kobalt, 5,1 Gew.-% Mangan, 1,9 Gew.-% Natrium, 3,1 Gew.-% Phosphor.

### Katalysator F (Vergleichskatalysator)

Die Herstellung erfolgte analog Katalysator A, jedoch wurden vor der Verstrangung zu je 4 kg Sprühpulver 73 g H₃PO₄ zugemischt. Der Katalysator wurde 1 h bei 650°C und danach 1 h bei 780°C calciniert. Man erhielt einen Katalysator mit einer spezifischen Oberfläche von 7,1 m²/g und folgender chemischer Zusammensetzung: 89,5 Gew.-% Kobalt, 5,1 Gew.-% Mangan, 1,41 Gew.-% Natrium, 4,1 Gew.-% Phosphor.

### Katalysator G (Vergleichskatalysator)

Die Herstellung erfolgte analog Katalysator F, jedoch ohne Vorcalcinierung 1 h bei 820°C. Man erhielt einen Katalysator mit einer spezifischen Oberfläche von 2,1 m²/g und folgender chemischer Zusammensetzung: 89,5 Gew.-% Kobalt, 5,1 Gew.-% Mangan, 1,4 Gew.-% Natrium, 4,2 Gew.-% Phosphor.

### Versuchsdurchführung

Ein vertikaler Rohrreaktor (Durchmesser: 16 mm, Füllhöhe: 50 cm, ölbeheizter Doppelmantel) wurde mit 400 g (200 ml) eines Kobalt-Vollkontaktes in Form von 4 mm-Strängen gefüllt. Zur Reduktion des Katalysators wurde drucklos unter Durchleiten von 200 Nl/h Wasserstoff die Temperatur innerhalb von 24 h schrittweise von 100 auf 340°C erhöht und dann 24 h bei 340°C gehalten.

Durch einen vor den Hydrierreaktor geschalteten Rohrreaktor (Durchmesser: 16 mm, Füllhöhe: 100 cm, ölbeheizter Doppelmantel), der mit 37 g (50 ml) Titandioxid in Form von 1,5 mm-Strängen gefüllt war, wurden bei einem Druck von 250 bar und einer Temperatur von 80°C stündlich von unten nach oben 80 g Isophoronnitril (Reinheit 99,0 %) und 270 g flüssiges Ammoniak gepumpt (Katalysatorbelastung 0,4 kg/l x h). Anschließend wurden stündlich 100 Nl/h (4,5 mol) Wasserstoff zugefahren und der Austrag aus dem vorgeschalteten Iminierungsreaktor bei einem Druck von 250 bar und einer Temperatur von 130°C von unten nach oben durch den Hydrierreaktor gefahren. Nach Entspannen auf Normaldruck wurde der Ammoniak abdestilliert und der Hydrieraustrag gaschromatographisch analysiert.

Die Ergebnisse sind in der nachfolgenden Tabelle zusammengestellt:

| Katalysator | Härte [N] nach Kochtest | Oberfläche [m²/g] | Aminonitril [ppm] | Ausbeute an Isophorondiamin [%] |
|---|---|---|---|---|
| A | 20 | 1,6 | 200 | 99 |
| B | 21 | 3,2 | < 100 | 99 |
| C | 11 | 15,1 | 200 | 99 |
| D | 19 | 3,0 | 200 | 97 |
| E | 15 | 6,2 | 400 | 99 |
| F | 0 | 7,1 | - | - |
| G | 1 | 2,1 | - | - |

## Patentansprüche

1. Kobaltkatalysatoren, deren katalytisch aktive Masse aus 55 bis 98 Gew.-% Kobalt, 0,2 bis 15 Gew.-% Phosphor, 0,2 bis 15 Gew.-% Mangan und 0,2 bis 15 Gew.-% Alkali, berechnet als Oxid, besteht, dadurch gekennzeichnet, daß man die Katalysatormasse in einem ersten Schritt bei Endtemperaturen von 550 bis 750°C und in einem zweiten Schritt bei Endtemperaturen von 800 bis 1000°C calciniert und die Kobaltkatalysatoren eine spezifische Oberfläche von größer oder gleich 1,5 m²/g aufweisen.

2. Kobaltkatalysatoren nach Anspruch 1, dadurch gekennzeichnet, daß deren katalytisch aktive Masse aus 75 bis 95 Gew.-% Kobalt, 0,5 bis 10 Gew.-% Phosphor, 2 bis 10 Gew.-% Mangan und 0,5 bis 5 Gew.-% Alkali, berechnet als Oxid, besteht.

3. Kobaltkatalysatoren nach Anspruch 1, dadurch gekennzeichnet, daß deren katalytisch aktive Masse aus 85 bis 95 Gew.-% Kobalt, 0,5 bis 5 Gew.-% Phosphor, 3 bis 7 Gew.-% Mangan und 0,5 bis 4 Gew.-% Alkali, berechnet als Oxid, besteht.

4. Kobaltkatalysatoren nach Anspruch 1, dadurch gekennzeichnet, daß man als Alkali Lithium, Natrium, Kalium und/oder Cäsium einsetzt.

5. Kobaltkatalysatoren nach Anspruch 1, dadurch gekennzeichnet, daß die Kobaltkatalysatoren nach Durchführung eines Kochtests zur Bestimmung der Basenstabilität eine Schneidhärte von größer oder gleich 10 Newton aufweisen.

6. Verfahren zur Hydrierung von organischen Nitrilen und/oder Iminen, dadurch gekennzeichnet, daß man Kobaltkatalysatoren nach Anspruch 1 einsetzt.

## Claims

1. A cobalt catalyst whose catalytically active composition comprises from 55 to 98 % by weight of cobalt, from 0.2 to 15 % by weight of phosphorus, from 0.2 to 15 % by weight of manganese and from 0.2 to 15 % by weight of alkali metal, calculated as oxide, wherein, in a first step, the catalyst composition is calcined at a final temperature of from 550 to 750°C and, in a second step, at a final temperature of from 800 to 1000°C and the cobalt catalyst has a specific surface area of greater than or equal to 1.5 m²/g.

2. A cobalt catalyst as claimed in claim 1, whose catalytically active composition comprises from 75 to 95 % by weight of cobalt, from 0.5 to 10 % by weight of phosphorus, from 2 to 10 % by weight of manganese and from 0.5 to 5 % by weight of alkali metal, calculated as oxide.

3. A cobalt catalyst as claimed in claim 1, whose catalytically active composition comprises from 85 to 95 % by weight of cobalt, from 0.5 to 5 % by weight of phosphorus, from 3 to 7 % by weight of manganese and from 0.5 to 4 % by weight of alkali metal, calculated as oxide.

4. A cobalt catalyst as claimed in claim 1, wherein the alkali metal used is lithium, sodium, potassium and/or cesium.

5. A cobalt catalyst as claimed in claim 1, which has a cutting hardness of greater than or equal to 10 newtons after carrying out an autoclave test for determining the base stability.

6. A process for hydrogenating organic nitriles and/or imines, wherein a cobalt catalyst as claimed in claim 1 is used.

## Revendications

1. Catalyseurs au cobalt, dont la masse catalytique active comprend 55 à 98 % en poids de cobalt, 0,2 à 15 % en poids de phosphore, 0,2 à 15 % en poids de manganèse et 0,2 à 15 % en poids d'alcali, calculés en tant qu'oxyde, caractérisé en ce que l'on calcine la masse du catalyseur dans une première étape à des températures finales de 550 à 750°C et dans une deuxième étape à des températures finales de 800 à 1000°C et les catalyseurs au cobalt présentent une surface spécifique supérieure ou égale à 1,5 m²/g.

2. Catalyseurs au cobalt selon la revendication 1, caractérisé en ce que leur masse catalytique active comprend 75 à 95 % en poids de cobalt, 0,5 à 10 % en poids de phosphore, 2 à 10 % en poids de manganèse et 0,5 à 5 % en poids d'alcali, calculés en tant qu'oxyde.

3. Catalyseurs au cobalt selon la revendication 1, caractérisé en ce que leur masse catalytique active comprend 85 à 95 % en poids de cobalt, 0,5 à 5 % en poids de phosphore, 3 à 7 % en poids de manganèse et 0,5 à 4 % en poids d'alcali, calculés en tant qu'oxyde.

4. Catalyseurs au cobalt selon la revendication 1, caractérisé en ce que l'on utilise en tant qu'alcali du lithium, du sodium, du potassium et/ou du césium.

5. Catalyseurs au cobalt selon la revendication 1, caractérisé en ce que les catalyseurs au cobalt présentent une dureté supérieure ou égale à 10 Newton, après la mise en oeuvre d'un test de cuisson pour la détermination de la stabilité vis à vis des bases.

6. Procédé d'hydrogénation de nitriles et/ou d'imines organiques, caractérisé en ce que l'on utilise les catalyseurs au cobalt selon la revendication 1.
